**Europäisches Patentamt**

**European Patent Office**

**Office européen des brevets**

(19)

(11) Publication number : **0 446 379 B1**

# EUROPEAN PATENT SPECIFICATION

(12)

(45) Date of publication of patent specification :
06.04.94 Bulletin 94/14

(51) Int. Cl.⁵ : **B01J 8/24, C07C 255/08, C07C 253/26**

(21) Application number : 90914763.9

(22) Date of filing : 04.10.90

(86) International application number :
PCT/JP90/01279

(87) International publication number :
WO 91/04961 18.04.91 Gazette 91/09

(54) **APPARATUS FOR PRODUCING ALPHA-BETA-UNSATURATED NITRILE.**

(30) Priority : 04.10.89 JP 257899/89
05.10.89 JP 258908/89

(43) Date of publication of application :
18.09.91 Bulletin 91/38

(45) Publication of the grant of the patent :
06.04.94 Bulletin 94/14

(84) Designated Contracting States :
DE ES FR GB IT NL

(56) References cited :
GB-A- 1 265 770

(73) Proprietor : Asahi Kasei Kogyo Kabushiki
Kaisha
2-6, Dojimahama 1-chome Kita-ku
Osaka-shi Osaka 530 (JP)

(72) Inventor : MUROYA, Hiroaki 2295,
Shimonagayacho Konan-ku
Yokohama-shi
Kanagawa 233 (JP)
Inventor : ISHII, Kanji 1073-18, Nishi-tomii
Kurashiki-shi
Okayama 710 (JP)
Inventor : OHTA, Masanobu 195-6, Kami-tomii
Kurashiki-shi
Okayama 710 (JP)
Inventor : TANAKA, Tetsuo 1319-27, Fukui
Nishi-tomii
Kurashiki-shi Okayama 710 (JP)

(74) Representative : Strehl Schübel-Hopf Groening
& Partner
Maximilianstrasse 54
D-80538 München (DE)

## Description

TECHNICAL FIELD

The present invention relates to an apparatus for manufacturing acrylonitrile or methacrylonitrile using propylene, isobutylene or tertiary butyl alcohol, and ammonia and an oxygen containing gas as starting materials.

BACKGROUND ART

Conventionally, a fluidized bed catalytic reactor is widely used when an $\alpha,\beta$-unsaturated nitrile is produced by the reaction of an olefin or a tertiary alcohol, and ammonia and an oxygen containing gas by a gas phase catalytic reaction.

Since the composition is within a range susceptible to explosion when gases fed to the reactor are mixed prior to the reaction, the mixed gas of olefin or tertiary alcohol and ammonia (hereinafter, referred to as "material gas") must be fed from a port separate from a port for feeding the oxygen containing gas.

According to Yoneichi Ikeda in Chemical Engineering 31, No. 10, 1013 (1970), it is well known that when the diameter of bubbles is made smaller in a fluidized bed, the contact efficiency between the gas and solid (i.e., catalyst) is improved. It is also known from the above literature that when the diameter of bubbles is made smaller in the synthesis of acrylonitrile by ammoxidation of propylene, the selectivity coefficient of acrylonitrile is improved.

In a conventional method, a packing in a reactor has been used to make the diameter of bubbles smaller, however, such a method is not preferable because it increases the cost of the apparatus.

When acrylonitrile or methacrylonitrile is produced by using a fluidized bed catalytic reactor, there is a problem in that the reactor cannot be operated for a long period since high boiling point substances produced by a side-reaction block the heat exchangers used for an air preheater, a boiler water preheater and the like disposed at the outlet of the reactor.

Further, when the reaction gas flow side of these heat exchangers begins to be blocked so that the pressure difference between the inlet and outlet of the heat exchangers is increased, the reaction pressure in the reactor is gradually increased. Since the catalyst used to make acrylonitrile or methacrylonitrile generally has a tendency to lower the yield of the acrylonitrile and methacrylonitrile when the reaction pressure is increased, an increase in the pressure difference in the heat exchangers and a resulting increase in the reaction pressure are not desirable even if the reactor is operated under normal conditions.

It is generally known that a so-called particle circulating flow is formed in a large fluidized bed reactor, in which a fluidized catalyst moves upwardly at the center of the reactor and moves downwardly in the vicinity of the wall thereof [Miyauchi et al., "Transport Phenomena and Reaction in Fluidized Catalyst Beds", Advances in Chem. Eng., Vol II, pp. 279 - 280, Academic Press (1981)].

The intensity of the circulating flow is known to be increased with an increase in the diameter of the reactor [Ibid., pp. 311 - 317, and Ben et al., "Reaction Engineering of Fluidized Bed", pp. 115 - 116, Baifukan, 1984].

A large fluidized bed reactor having a radius exceeding 3 m has a serious drawback in that the contact efficiency with a fluidized catalyst used therein is lowered due to a so-called reverse mixing by which the produced substance is mixed with "material gas", a phenomenon wherein the "material gas" is fed to the reactor without being reacted, and the like, resulting in a decrease in the reaction yield and thus the catalyst cannot perform to its full capability, because the so-called particle circulating flow, which is caused by the catalyst moving upwardly at the center of the reactor and moving downwardly in the vicinity of the wall thereof, is intensive in the fluidized bed reactor. To cope with this drawback, bubbles are conventionally redispersed by the provision of a structure such as a perforated plate or the like to improve fluidization. Nevertheless, the redispersion of the bubbles effected by the perforated plate or the like is not preferable, because the pressure loss is increased at the perforated plate or a sufficient amount of catalyst particles does not fall down, which couses the formation of a reaction gas accumulation region at the lower portion of the inside structure, by which the yield of the target product is lowered.

As is well known, it is especially critical that the "material gas" and oxygen containing gas are reacted in a fluidized bed reactor after they are promptly mixed into a uniform gas mixture.

United State Patent No. 4,801,731 proposes a method of mixing oxygen containing gas and propylene/ ammonia mixed gas by a counter flow, when acrylonitrile is made, in such a manner that oxygen containing gas blowoff pipes are disposed in alignment with propylene/ ammonia mixed gas blowoff pipes.

Nevertheless, with the large fluidized bed reactor having a diameter exceeding 3 m which is usually commercially used, the existence of a circulating flow of catalyst particles is prominent, so that the oxygen containing gas jet at the outer circumference of the reactor is curved toward the center thereof by the effect of a downward particle circulating flow in the vicinity of the wall of the reactor. As a result, when the large reactor having a diameter of 3 m or more is used, the method disclosed in United State Patent No. 4,801,731 has a drawback in that "material gas" is not sufficiently mixed with oxygen containing gas in the outer circumference, with the result that an yield of α,β-unsaturated nitrile is lowered in the reactor as a whole.

A reactor for the use in the production of unsaturated aldehydes and acids from olefins and oxygen or the production of unsaturated nitriles from olefins, oxygens and ammonia is disclosed in GB-A-1 265 770. Said reactor comprises a housing, a distributor plate mounted transversally in the lower portion of the housing and joined at its periphery to the wall of the housing, means for introducing a fluid reactant into the housing below the distributor plate, a conduit within the housing located above and adjacent the peripheral area of the distributor plate and having a plurality of orifices facing the said area, means for introducing a fluid into the conduit and means for removing reactor effluent from the upper portion of the housing.

DISCLOSURE OF THE INVENTION

Taking the above into consideration, the inventors have achieved the present invention, as a result of the improvement of a reactor made by paying attention to the diameter of the produced bubbles and the catalyst circulating flow. Therefore, an object of the present invention is to provide a fluidized bed reactor which can improve the yield of α,β-unsaturated nitrile as a whole in such a manner that the yield of the α,β-unsaturated nitrile is improved the reactor can be continuously operated for a long period, and the reaction yield from olefin or tertiary butyl alcohol is improved in the outer circumference of the reactor.

More specifically, the inventors have achieved the present invention by finding that the above objects are achieved by an α,β-unsaturated nitrile manufacturing apparatus comprising a fluidized bed reactor, which comprises:

(1) a "material gas" feeder (hereinafter, referred to as MGF) horizontally disposed at a lower portion of a reactor and having a plurality of "material gas" blowoff nozzles (A) disposed on the lower surface thereof at substantially the same intervals, the "material gas" feeder preferably having a diameter a little smaller than that of the reactor, and the "material gas" blowoff nozzles being directed downwardly of the reactor; and

(2) an oxygen containing gas feeder (hereinafter referred to as OGF) disposed horizontally, spaced apart from the above MFG at a predetermined interval, preferably having the same diameter as that of the reactor, and having oxygen containing gas blowoff pipes (B) disposed on the upper surface thereof in opposing relation with the above nozzles (A), the number of the pipes (B) being the same as that of the nozzles (A), and, in particular, the reactor is arranged such that:

(i) the intervals between the extreme end of the nozzles (A) and the extreme end of the pipes (B) are set to 25 to 300 mm;
(ii) the intervals between a plurality of the pipes (B) are set to 90 mm to 250 mm, and the number of the pipes is 16 to 123 pieces/m$^2$ in the cross sectional area of the reactor; and
(iii) the pipes (B) at the outermost portion are arranged such that the intervals between the inside wall of the reactor and the pipes (B) nearest to the inside wall are set to 300 mm.

Further, the inventors have found that the nozzles (A) located at the outer circumference of the MGF are not aligned with the pipes (B) in opposing relation therewith, but are slightly offset toward the center of the reactor with respect to the pipes (B), enables the reactor to be operated for a long period with a small amount of a by-product having a high boiling temperature and without lowering the yield at the outer circumference, particularly when the reactor has a large size.

BRIEF DESCRIPTION OF THE DRAWINGS

Figure 1 is a cross sectional view of a main part of a fluidized bed reactor showing an embodiment of the present invention;
Figure 2 is a schematic view showing the relative position of an oxygen containing gas blowoff pipe of an oxygen containing gas feeder and "material gas" blowoff nozzles of a "material gas" feeder, and the flow of catalyst particles and oxygen containing gas in an embodiment of the present invention;

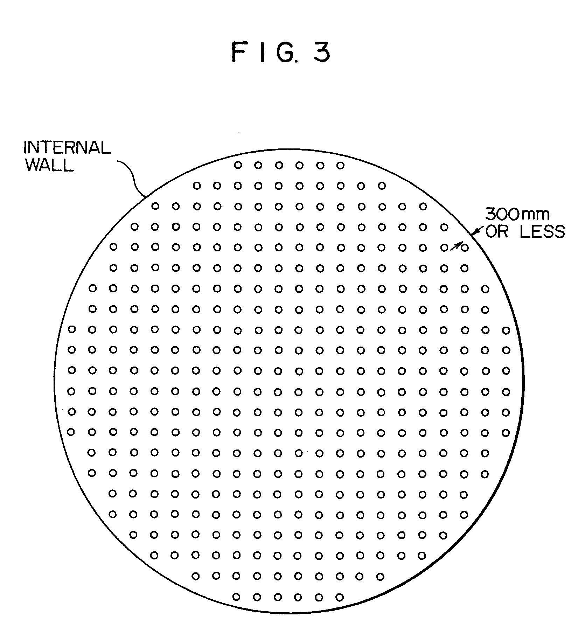

Figure 3 shows a plane view of the oxygen containing gas feeder; and

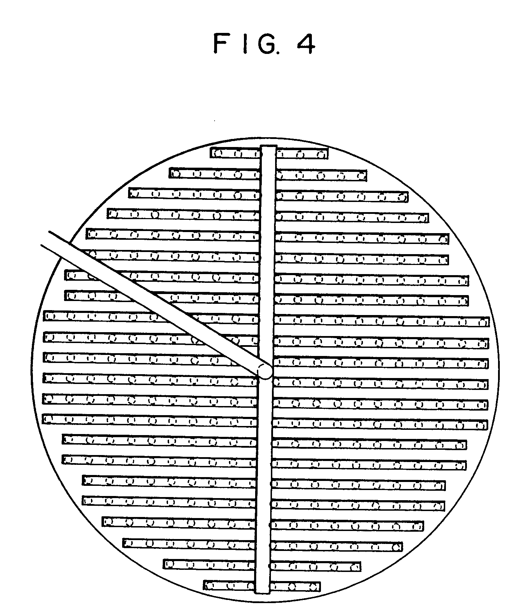

Figure 4 is a plane view of the "material gas" feeder. In the drawings, 1 designates a fluidized bed reactor main body, 2 designates a heat removing coil, 3 designates the "material gas" feeder, 4 designates the oxygen containing gas feeder, 5 designates the blowoff nozzles of the "material gas" feeder, 6 designates the blowoff pipes of the oxygen containing gas feeder, 7 designates an oxygen containing gas jet formed by the blowoff pipes of the oxygen containing gas feeder, 8 designates a catalyst particle circulating flow,

and θ designates a "relative angle".

## BEST MODE OF CARRYING OUT THE PRESENT INVENTION

The present invention relates to an α,β-unsaturated nitrile manufacturing apparatus for manufacturing an α,β-unsaturated nitrile having the same number of carbons as in a starting material which is an olefin or tertiary butyl alcohol by causing propylene, isobutylene or tertiary butyl alcohol, and ammonia and an oxygen containing gas to be reacted, comprising:

a reactor;

a "material gas" feeder horizontally disposed at a lower portion of the reactor and having a plurality of "material gas" blowoff nozzles disposed on the lower surface thereof; and

an oxygen containing gas feeder disposed at a lower portion of the "material gas" feeder in parallel therewith, spaced apart therefrom at a predetermined interval and having oxygen containing gas blowoff pipes disposed on the upper surface of the oxygen containing gas feeder in opposing relation with the "material gas" blowoff nozzles, the number of the oxygen containing gas blowoff pipes being the same as that of the "material gas" blowoff nozzles provided on the "material gas" feeder;

wherein the predetermined interval is 25 to 300 mm as measured by the respective intervals between the nozzles and the corresponding pipes; and

the intervals between the oxygen containing gas blowoff pipes are 90 to 250 mm, and the number of the pipes is 16 to 123 pieces/m² in the cross sectional area of the reactor. Said apparatus is characterized in that the series of said raw material gas blowoff nozzles disposed in the portions of said reactor having a ratio of the distance from the center of the reactor (r) to the circumference of the reactor (R) r/R of 0.8 to 1.0 are offset towards the center of said reactor such that the angle θ formed by the line connecting the center of the extreme end of the oxygen containing gas blowoff pipe and the center of the extreme end of the opposing raw material gas blowoff nozzle and the vertical line passing through the center of the extreme end of the oxygen containing gas blowoff pipe is 40° or less.

Another aspect of the present invention relates to an α,β-unsaturated nitrile manufacturing apparatus, wherein the intervals between the series of oxygen containing gas blowoff pipes nearest to the inside wall of the reactor and the inside wall are set to 300 mm or less.

The catalyst used for the reaction can be selected from any of the ammoxidation catalysts usually employed to make acrylonitrile or methacrylonitrile, and, for example, catalysts disclosed in Japanese Patent Kokoku (Post Exam. Publication) Nos. Sho 36-5870, Sho 38-17967, Sho 59-50667, Sho 61-58462, United States Patent No. 4,495,109, and the like can be used to make acrylonitrile, and catalysts disclosed in Japanese Patent Kokoku Nos. Hei 1-34221, Hei 1-34222, and the like can be used to make methacrylonitrile.

When acrylonitrile and methacrylonitrile are produced, the reaction is carried out at a reaction temperature in the range of 400°C to 500°C and at a reaction pressure in the range of 0.2 Kg/cm² G to 2 Kg/cm² G. Propylene, isobutylene or tertiary butyl alcohol, and ammonia are mixed in a molar ratio of 1.0 to 1.3 so that the amount of propylene, isobutylene or tertiary butyl ealcohol is equal to or slightly in excess of the amount of ammonia on a molar basis.

The oxygen containing gas must contains oxygen in a molecular state, and, for example, pure oxygen or pure oxygen diluted by an inert gas such as nitrogen gas is used, and, in particular, air is preferably used.

When air is used as the oxygen containing gas, the amount of air to be fed to the reactor must be in the range from 7 to 14 times, on a molar basis, the amount of propylene, isobutylene, or tertiary butyl alcohol.

The intervals between the oxygen containing gas blowoff pipes (B) are in the range of 90 to 250 mm. When, however, the intervals between the blowoff pipes (B) are too narrow, the efficiency is reduced, because produced air bubbles coming from the adjacent blowoff pipes come into contact with each other and grow into larger air bubbles.

The intervals between the blowoff pipes (B) may be or may not be fixed. The blowoff pipes may be arranged in a square, rectangular or triangular pattern.

The density of the oxygen containing gas blowoff tubes (B) is in the range of 16 to 123 pieces/m² in the horizontal cross sectional area of the reactor.

The diameter of air bubbles produced on the oxygen containing gas feeder or in the blowoff pipes (B) can be determined from the following equations [Miwa et al., Chemical Engineering, 35, 770 (1971)].

$$Db0 \ = \ 1.38 \ g^{-0.2} \ (At/Nor)^{0.4}(Uo \ - \ Umf)^{0.4} \quad (1)$$

$$Db0 \ = \ 3.77 \ g^{-1} \ (Uo \ - \ Umf)^2 \quad (2)$$

The diameter of the produced bubbles is the larger one of the values determined by the equations (1) and (2).

Db0     : diameter of produced bubbles [m]

Uo      : gas velocity in the reactor [m/sec]
Umf     : minimum fluidization velocity [m/sec]
At      : reactor cross sectional area [$m^2$]
Nor     : number of blowoff pipes [pieces]
g       : gravitational acceleration [$m/sec^2$]

The diameter of produced air bubbles is calculated as follows, assuming that the blowoff pipes are arranged in a square pattern and Uo = 0.5 m/sec.

| | | |
|---|---|---|
| Intervals between Adjacent Blowoff Pipes [mm] | 90 | ·250 |
| Number of Blowoff Pipes per 1 $m^2$ (Nor/At) [pieces/$m^2$] | 123 | 16 |
| Db0 obtained by Equation (1) [mm] | 97 | 220 |
| Db0 obtained by Equation (2) [mm] | 96 | 96 |

Intervals between the blowoff pipes less than 90 mm are not preferable, because the diameter of air bubbles then is made larger than the intervals between the blowoff pipes, causing the air bubbles to come into contact with each other and grow into larger air bubbles, whereas intervals between the blowoff pipes exceeding 250 mm are also not preferable, because the diameter of the air bubbles greatly exceeds 200 mm. Therefore, intervals between the blowoff pipes are preferably selected to be in the range of 90 mm to 250 mm.

The oxygen containing gas blowoff pipe may or may not have a uniform inside diameter. However, the extreme end of the oxygen containing gas blowoff pipe is preferably narrowed down to prevent the catalyst from entering the oxygen containing gas feeder.

Oxygen containing gas is preferably blown off from the oxygen containing gas blowoff pipes at a speed in the range of 10 m/sec to 80 m/sec, and more preferably in the range of 30 m/sec to 60 m/sec. An excessively high blow off speed is not preferable, because it causes loss of the catalyst.

The "material gas" blowoff nozzle may or may not have a uniform nozzle diameter.

"Material gas" is preferably blown off from the blowoff nozzles at a speed in the range of 10 m/sec to 80 m/sec, and more preferably in the range of 30 m/sec to 60 m/sec. An excessively high blowoff speed is not preferable, because it causes loss of the catalyst.

The number of the "material gas" blowoff nozzles is preferably the same as that of the oxygen containing gas blowoff pipes, the relative position therebetween is preferably in an opposing relationship, and the relative intervals therebetween (hereinafter, referred to as "relative intervals") are preferably 25 to 300 mm. When the "relative intervals" are excessively short, the "material gas" blowoff nozzles or the oxygen containing gas blowoff pipes may be damaged by melting due to an abnormal reaction, whereas when the "relative intervals" are excessively long, "material gas" is not sufficiently mixed with oxygen containing gas, and thus the yield of $\alpha,\beta$-unsaturated nitrile is lowered.

In the description and claims of the present invention, the term "outer circumference" refers to the portions of the reactor having a ratio (hereinafter, referred to as r/R) in the range of 0.8 to 1.0 of the distance from the center of the reactor to the circumference of the reactor.

To restrict the circulating flow of catalyst particles at the outer circumference of the reactor and to cause the "material gas" and oxygen containing gas to be sufficiently mixed, the downward flow of the fluidized catalyst in the vicinity of the inside wall of the reactor is restricted in such a manner that the intervals between the inside wall of the reactor and the oxygen containing gas blowoff pipes nearest to the inside wall (hereinafter, referred to as intervals to wall) are preferably set to 300 mm or less, and more preferably to 50 to 200 mm, and further the "material gas" blowoff nozzles, which are located in the outer circumference, are offset toward the center of the reactor with respect to the oxygen containing gas blowoff pipes disposed at the lower portion of the "material gas" blowoff nozzles in opposing relation therewith. With this arrangement, the conversion yield of propylene, isobutylene or tertiary butyl alcohol is greatly improved, and the yield of acrylonitrile or methacrylonitrile is improved in the reactor as a whole.

5

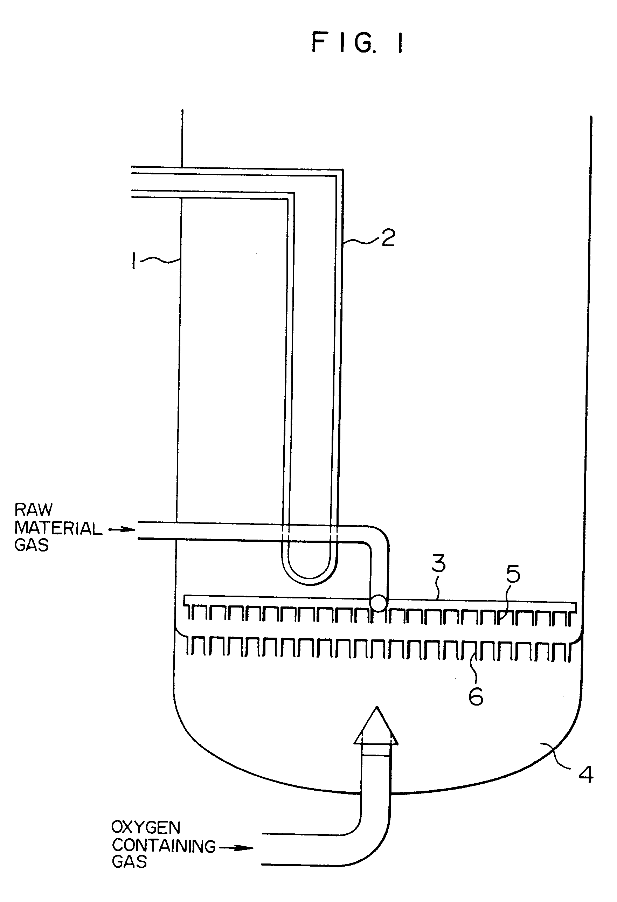

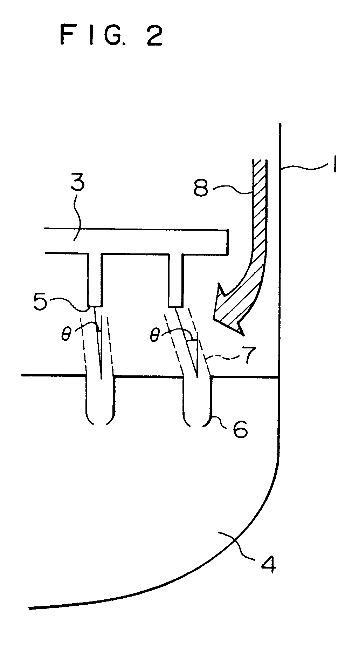

Next, an example of an apparatus according to the present invention will be described with reference to Figures 1 and 2. Figure 1 is an example of a fluidized bed reactor used in the present invention, wherein 1 designates a fluidized bed reactor main body, 2 designates a heat removing coil, 3 designates a "material gas" feeder (MGF), 4 designates an oxygen containing gas feeder (OGF). Figure 2 shows an example of a concept of the relative positional relationship of the oxygen containing gas blowoff pipes (B) and the "material gas" blowoff nozzles (A), and a circulating flow of catalyst particles, wherein 5 designates a "material gas" blowoff nozzle, 6 designates an oxygen containing gas blowoff pipe, 7 designates a jet stream formed by the oxygen containing gas blowoff pipe, and 8 designates the circulating flow of catalyst particles.

Further, the degree by which the "material gas" blowoff nozzles, which are located in the outer circumference, are offset toward the center of the reactor with respect to the oxygen containing gas blowoff pipes disposed at the lower portion thereof in opposing relation therewith depends on the intensity of the circulating flow of catalyst particles and the relative intervals between the "material gas" blow off nozzles and the oxygen containing gas blowoff pipes, and the "material gas" blowoff nozzles are preferably offset toward the center of the reactor such that the angle, which is formed by the line connecting the center of the extreme end of the oxygen containing gas blowoff pipe and the center of the extreme end of the "material gas" blowoff nozzle and the vertical line passing through the center of the extreme end of the oxygen containing gas blowoff pipe (hereinafter, referred to as a "relative angle $\theta$"), is 40° or less, and preferably 30° or less.

The present invention will be described below with reference to Comparative Examples and Examples, but the scope of the present invention is not limited to these examples. Note that the density of a fluidized catalyst bed was determined by a generally known pressure difference using pressure taps located at the positions 750 mm and 1250 mm above the oxygen containing gas blowoff pipes for measuring the static pressure difference, the locations of the pressure taps satisfying the equation $r/R = 0.0$ at the center of the fluidized bed reactor and satisfying the equation $r/R = 0.9$ in the outer circumference, wherein the driving force by which the fluid of circulating particles is formed is the difference in density in a radial direction of the fluidized bed [See "Fluidization Engineering" (D. Kunii, O. Levenspiel), P. 354]. Thus, it is apparent that when the density of the fluidized catalyst bed is uniform in a radial direction, the flow of circulating particles is not formed. Further, unreacted olefin was analyzed by a gas chromatograph in such a manner that gas sampling nozzles were disposed at the center at a height of 9 m satisfying the equation $r/R = 0.0$ and in the outer circumference at the same height satisfying the equation $r/R = 0.9$ and gas coming from the nozzles was washed with water. The usual instruments and attachments were used therein.

Comparative Example 1

An oxygen containing gas feeder was disposed at a lower portion of a fluidized bed reactor having an inside diameter of 3.7 m, and blowoff pipes were arranged in a square having a side of 300 mm. A "material gas" feeder was disposed at the upper portion of the fluidized bed reactor, the feeder being a pipe grid type, arranged in a square having a side of 300 mm, and having blowoff nozzles for blowing off gas downwardly.

The number of oxygen containing gas blowoff pipes was the same as that of the "material gas" blowoff nozzles, and the "material gas" blowoff nozzles were disposed 100 mm just above the opposing oxygen containing gas blowoff pipes. The "relative angle $\theta$" of the outer circumference was set to $0^0$ and "intervals to wall" were set to 150 mm.

The inside diameter of the oxygen containing gas blowoff nozzle was uniform and determined such that the blowing off speed of gas therefrom was 46 m/sec.

The inside diameter of the "material gas" blowoff nozzle was uniform and determined such that the blowing off speed of gas therefrom was 40 m/sec.

The reactor was filled with a fluidized bed catalyst in an amount of 25 tons composed of molybdenum-bismuth-iron carried on silica.

Air was fed to the oxygen containing gas feeder in an amount of 9,100 Nm$^3$/h, 96 mole% purified propylene in an amount of 1,000 Nm$^3$/h and ammonia in an amount of 1,150 Nm$^3$/h were fed to the "material gas" feeder, and they were reacted at a reaction temperature of 470°C. Table 1 shows the result of the reaction obtained after 2 weeks from the start of the reaction.

The definitions of conversion and high boiling point substance are as follows.

conversion (%) = (weight of reacted propylene/weight of fed propylene) x 100

yield (%) = (weight of carbon in product/weight of carbon in fed propylene) x 100

high boiling point substance: total of acetic acid, acrylic acid, fumaronitrile, 3-cyano-pyridine, and "others", wherein the weight of carbon in the "others" was calculated from the total area of the peaks other than those of acetic acid, acrylic acid, fumaronitrile, and 3-cyano-pyridine on a gas chromatograph using the conversion factor of fumaronitrile.

Gas Chromatography:

| | |
|---|---|
| column : | made of glass 3 mm x 3 m |
| filler : | FON of Wako Junkayu K.K. |
| | 10 %/Shimalite TPA |
| column temperature : | 160°C |

The reaction was interrupted after 4.2 months, because the reaction pressure of the reactor was increased with an increase in a pressure difference on the reacted gas side of an air preheater (not shown) and a boiler water preheater (not shown) provided at the outlet of the reactor, and thus it was difficult to feed the material gas and the air to the feeders.

Examples 1 - 4

Reactors similar to that used in Comparative Example were used except that the blowoff pipes were arranged in a square having a side of a different length, respectively. The same catalyst and reacting conditions as those of the Comparative Example were used. The blowoff pipes of the reactor of Example 3, however, were arranged in a rectangular pattern having a short side of 90 mm and a long side of 180 mm. Table 1 shows the result of the reaction obtained after 2 weeks from the start of the reaction.

Table 1

| | Comparative Example 1 | Example 1 | Example 2 | Example 3 | Example 4 |
|---|---|---|---|---|---|
| Arrangement of Blowoff Nozzles and Pipes | Square | Square | Square | Rectangle | Square |
| Arrangement Pitch (mm) | 300 x 300 | 180 x 180 | 140 x 140 | 90 x 180 | 90 x 90 |
| Number of Blowoff Pipes (pieces/m²) | 11 | 31 | 51 | 62 | 123 |
| Number of Blowoff Nozzles (pieces/m²) | 11 | 31 | 51 | 62 | 123 |
| Conversion of Propylene (%) | 98.5 | 99.1 | 99.3 | 99.2 | 99.1 |
| Yield of Acrylonitrile (%) | 76.5 | 77.8 | 78.2 | 77.6 | 77.2 |
| Yield of High Boiling Point Substance (%) | 2.8 | 1.4 | 1.1 | 1.6 | 1.4 |
| Continuous Operation Period of Reactor (month) | 4.2 | 11* | 11* | 11* | 11* |

* The operation was interrupted, not because the heat exchangers at the outlet of the reactor were blocked, but because high pressure equipments of a plant were inspected by regulations.

EP 0 446 379 B1

Comparative Examples 2

An oxygen containing gas feeder was disposed at a lower portion of a fluidized bed reactor having an inside diameter of 7.8 m and blowoff pipes were arranged in a square pattern having a side of 160 mm. A "material gas" feeder was disposed at the upper portion of the fluidized bed catalytic reactor, the feeder being a pipe grid type, arranged in a square having a side of 160 mm and having blowoff nozzles for blowing gas downwardly. The number of the oxygen containing gas blowoff pipes was the same as that of the "material gas" blowoff nozzles and the "relative intervals" were set to 75 mm. The "relative angle $\theta$" of the outer circumference was set to 0°, and the "intervals to wall" were set to 500 mm.

The reactor was filled with a molybdenum-bismuth-iron catalyst carried on silica having a particle size of 10 to 100 $\mu$m and an average particle size of 50 $\mu$m so that the static bed height was 3 m, air in an amount of 41,000 Nm$^3$/h was fed to the oxygen containing gas feeder, 96 mole% purified propylene in an amount of 4,000 Nm$^3$/h and ammonia in an amount of 4,800 Nm$^3$/h were fed to the "material gas" feeder, and they were subjected to reaction at a reaction temperature of 450°C and a pressure of 1 Kg/cm$^2$G. Table 2 shows the result of the reaction.

Examples 5 to 7

The reaction was carried out in a reactor similar to that of Comparative Example 2 under the same reacting conditions as those of Comparative Example 2 using an oxygen containing gas feeder and a "material gas" feeder similar to those of Comparative Example 2 except that the "intervals to wall" and "relative angle $\theta$" were set to the values shown in Table 2. As a result, the circulating flow of catalyst particles and the amount of unreacted propylene in the outer circumference were greatly reduced, as shown in Table 2, and thus the conversion of propylene and the yield of acrylonitrile were improved in the reactor as a whole.

Example 8

An oxygen containing gas feeder was disposed at a lower portion of a fluidized bed reactor having an inside diameter of 5.3 m and blowoff pipes were arranged in a square pattern having a side of 180 mm. A "material gas" feeder was disposed at the upper portion of the fluidized bed reactor, the feeder being a pipe grid type, arranged in a square pattern having a side of 180 mm, and having blowoff nozzles for blowing gases downwardly. The number of oxygen containing gas blowoff pipes was the same as that of the "material gas" blowoff nozzles, and the "relative intervals" between the oxygen containing gas blowoff pipes disposed at the portions other than the outer circumference and the "material gas" blowoff nozzles in opposing relation therewith were set to 100 mm. The "relative angle $\theta$" of the outer circumference was set to 15°, and "intervals to wall" were set to 120 mm. The reactor was filled with a molybdenum-bismuth-iron catalyst carried on silica having a particle size of 10 to 100 $\mu$m and an average particle size of 50 $\mu$m so that the static bed height was 3 m.

Air in an amount of 28,000 Nm$^3$/h was fed to the oxygen containing gas feeder, isobutylene in an amount of 2,300 Nm$^3$/h and ammonia in an amount of 3,000 Nm$^3$/h were fed to the "material gas" feeder, and they were subjected to reaction at a reaction temperature of 430°C and a pressure of 1 Kg/cm$^2$G. Table 2 shows the result of the reaction.

Table 2

| Example | "Intervals to Wall" [mm] | "Relative Angle θ" of Outer Circumference [deg] | Fluidized Catalyst Density | | Unreacted Propylene or Isobutylene Concentration | | Conversion of Propylene in Entire Reactor (%) | Yield of Acrylonitrile in Entire Reactor (%) |
|---|---|---|---|---|---|---|---|---|
| | | | Center [Kg/m3] | Outer Circumference [Kg/m3] | Center [Vol.%] | Outer Circumference [Vol.%] | | |
| Comparative Example 2 | 500 | 0 | 480 | 730 | 0.17 | 1.05 | 98.3 | 77.2 |
| Example 5 | 150 | 15 | 570 | 590 | 0.09 | 0.11 | 99.6 | 78.7 |
| Example 6 | 150 | 0 | 560 | 575 | 0.10 | 0.17 | 99.3 | 78.3 |
| Example 7 | 300 | 27 | 470 | 700 | 0.08 | 0.20 | 98.7 | 77.8 |
| Example 8 | 120 | 15 | 560 | 570 | 0.07 | 0.08 | – | – |

The effect of this apparatus will be apparent from the result shown in Table 2.

Example 6 shows that the "intervals to wall" set to 150 mm enable the yield of acrylonitrile to be improved in the reactor as a whole as compared with Comparative Example 2 by reducing unreacted propylene in the outer circumference.

Further, the results of Examples 5 and 6 show that when the "intervals to wall" are set to the same value, a "relative angle θ" is preferably provided. More specifically, the efficiency of setting a "relative angle θ" of the outer circumference to 15° is observed in Example 5 as compared with Example 6. Further, as shown in Example 7, it is apparent that when the "intervals to wall" are large, efficiency can be obtained by increasing the "relative angle θ".

Example 9

The reaction was carried out using a reactor and catalyst similar to those of Example 8. Air in an amount of 24,000 Nm³/h was fed to the oxygen containing gas feeder, 84 wt% purified tertiary butyl alcohol aqueous solution in an amount of 3,600 Nm³/h in a gas phase and ammonia in an amount of 2,600 Nm³/h were fed to the "material gas" feeder, and they were subjected to reaction at a reaction temperature of 440°C and a pressure of 1 Kg/cm². The result of the reaction is as follows.

|  | Central portion | Outer circumference |
|---|---|---|
| fluidized catalyst density (Kg/m3) | 580 | 600 |
| unreacted isobutylene (vol%) | 0.10 | 0.13 |

Note)   Since tertiary butyl alcohol is converted into isobutylene and water at once in the reactor, unreacted isobutylene is detected.

INDUSTRIAL APPLICABILITY

The apparatus of the present invention presents an excellent industrial advantage in that the yield of $\alpha,\beta$-unsaturated nitrile is not lowered by the reaction gas staying region formed at the lower portion of the inside structure of the reactor due to an increased pressure loss at the portion where a structure such as packing, a perforated plate, or the like is provided in the reactor or because a sufficient number of catalyst particles does not fall down, the yield of the $\alpha,\beta$-unsaturated nitrile is increased by the simply arranged apparatus, and further the reactor can be continuously operated for a long period without blocking the heat exchangers provided at the outlet of the reactor.

**Claims**

1.   An apparatus for manufacturing an $\alpha,\beta$-unsaturated nitrile by reacting as the raw material an olefin or tertiary butyl alcohol with ammonia and oxygen containing air to produce an $\alpha,\beta$-unsaturated nitrile having the same number of carbon atoms as the raw material, said apparatus comprising:

a reactor;

a raw material gas feeder horizontally disposed at the lower portion of said reactor and having a plurality of raw material gas blowoff nozzles disposed on the lower surface thereof; and

an oxygen containing gas feeder disposed below said raw material gas feeder and parallel therewith, spaced apart therefrom at a predetermined interval and having oxygen containing gas blowoff pipes disposed on the upper surface of said oxygen containing gas feeder in opposing relationship with said raw material gas blowoff nozzles, the number of said oxygen containing gas blowoff pipes being the same as the number of said raw material gas blowoff nozzles provided on said raw material gas feeder;

wherein said predetermined interval is 25 to 300 mm as measured by the respective intervals between said nozzles and said pipes;

wherein the intervals between said oxygen containing gas blowoff pipes are 90 to 250 mm and the number of said pipes is 16 to 123 pieces/m$^2$ in the cross sectional area of said reactor, and wherein the series of said raw material gas blowoff nozzles disposed in the portions of said reactor having a ratio of the distance from the center of the reactor (r) to the circumference of the reactor (R) r/R of 0.8 to 1.0 are offset towards the center of said reactor such that the angle θ formed by the line connecting the center of the extreme end of the oxygen containing gas blowoff pipe and the center of the extreme end of the opposing raw material gas blowoff nozzle and the vertical line passing through the center of the extreme end of the oxygen containing gas blowoff pipe is 40° or less.

2.   A manufacturing apparatus according to claim 1, wherein the intervals between the series of said oxygen containing gas blowoff pipes nearest to the inside wall of said reactor and said inside wall are 300 mm or less.

**Patentansprüche**

1.   Vorrichtung zur Herstellung eines α,β-ungesättigten Nitrils durch Umsetzung eines Olefins oder von tertiärem Butylalkohol als Rohmaterial mit Ammoniak und Sauerstoff enthaltender Luft, wobei ein α,β-ungesättigtes Nitril mit der gleichen Anzahl von Kohlenstoffatomen wie das Rohmaterial gebildet wird, wobei die Vorrichtung umfaßt :

einen Reaktor,

eine Beschickungseinrichtung für Rohmaterialgas, die im unteren Teil des Reaktors horizontal angeordnet ist und die zahlreiche Ausblasdüsen für das Rohmaterialgas aufweist, die an ihrer unteren Oberfläche vorgesehen sind, und

eine Beschickungseinrichtung für Sauerstoff enthaltendes Gas, die unterhalb der Beschickungseinrichtung für das Rohmaterialgas und parallel zu dieser in einem vorbestimmten Abstand von dieser entfernt ist, wobei Ausblasrohre für das Sauerstoff enthaltende Gas an der oberen Oberfläche der Beschickungseinrichtung für das Sauerstoff enthaltende Gas gegenüber von den Ausblasdüsen für das Rohmaterialgas angeordnet sind, wobei die Anzahl der Ausblasrohre für das Sauerstoff enthaltende Gas die gleiche ist wie die Anzahl der auf der Beschickungseinrichtung für das Rohmaterialgas vorgesehenen Ausblasdüsen für das Rohmaterialgas,

wobei der vorbestimmte Abstand 25 bis 300 mm, gemessen als Abstand zwischen den Düsen und den Rohren ist,

wobei die Zwischenräume zwischen den Ausblasrohren für das Sauerstoff enthaltende Gas 90 bis 250 mm und die Anzahl der Rohre 16 bis 123 Stück/m$^2$ in der Querschnittsfläche des Reaktors sind, und wobei die Reihe der Ausblasdüsen für das Rohmaterialgas, die in den Bereichen des Reaktors angeordnet sind, in denen das Verhältnis des Abstands vom Zentrum des Reaktors (r) zu dem Umfang des Reaktors (R) r/R 0,8 bis 1,0 beträgt, derart in Richtung zum Zentrum des Reaktors geneigt sind, daß der Winkel θ, der zwischen der Linie, die den Mittelpunkt des äußeren Endes der Ausblasrohrs für das Sauerstoff enthaltende Gas und den Mittelpunkt des äußeren Endes der gegenüberliegenden Ausblasdüse für Rohmaterialgas verbindet und der vertikalen Linie, die durch den Mittelpunkt des äußeren Endes des Ausblasrohrs für das Sauerstoff enthaltende Gas verläuft, 40° oder weniger beträgt.

2.   Herstellungsvorrichtung nach Anspruch 1, in der die Zwischenräume zwischen der Reihe der Ausblasrohre für das Sauerstoff enthaltende Gas, die der Innenwandung des Reaktors am nächsten sind, und dieser Innenwandung 300 mm oder weniger betragen.

**Revendications**

1.   Installation de production d'un nitrile insaturé en α,β par réaction comme matière première d'une oléfine ou d'alcool butylique tertiaire sur de l'air contenant de l'ammoniac et de l'oxygène, pour préparer un nitrile insaturé en position α,β ayant le même nombre d'atomes de carbone que la matière première, l'installation comprenant :

un réacteur;

un dispositif de chargement de la matière première gazeuse, disposé horizontalement à la partie inférieure du réacteur et ayant plusieurs buses d'insufflation de matière première gazeuse, disposées sur sa surface inférieure; et

un dispositif de chargement de gaz contenant de l'oxygène, disposé en-dessous du dispositif de chargement de matière première gazeuse et parallèlement à celui-ci, à distance de celui-ci à un intervalle prescrit et ayant des conduits d'insufflation de gaz contenant de l'oxygène disposés à la surface supérieure du dispositif de chargement de gaz contenant de l'oxygène en opposition avec les buses d'insufflation de matière première gazeuse, le nombre de conduits d'insufflation de gaz contenant de l'oxygène étant le même que celui des buses d'insufflation de matière première gazeuse prévues sur le dispositif de chargement de matière première gazeuse;

l'intervalle prescrit étant compris entre 25 et 300 mm, tel que mesuré par les intervalles respectifs entre les buses et les conduits;

les intervalles entre les conduits d'insufflation de gaz contenant de l'oxygène étant compris entre 90 et 250 mm, et le nombre des conduits étant compris entre 16 et 123 pièces/m² dans la section transversale du réacteur,

les rangées de buses d'insufflation de matière première gazeuse disposées dans les parties du réacteur ayant un rapport de la distance au centre du réacteur (r) à la circonférence du réacteur (R) r/R de 0,8 à 1,0, étant décalées vers le centre du réacteur de manière que l'angle θ formé par la droite, passant par le centre de l'extrémité extrême du conduit d'insufflation de gaz contenant de l'oxygène et par le centre de l'extrémité extrême de la buse d'insufflation de matière première gazeuse opposée, et la verticale, passant par le centre de l'extrémité extrême du conduit d'insufflation de gaz contenant de l'oxygène, soit de 40° ou soit inférieur à 40°.

2.	Installation suivant la revendication 1, dans laquelle les intervalles entre les rangées de conduits d'insufflation de gaz contenant de l'oxygène les plus proches de la paroi intérieure du réacteur et la paroi du réacteur, sont de 300 mm ou sont inférieurs à 300 mm.

# F I G. 1

RAW
MATERIAL →
GAS

OXYGEN
CONTAINING →
GAS

F I G. 2

# F I G. 3

INTERNAL
WALL

300mm
OR LESS

F I G. 4